# EUROPÄISCHE PATENTSCHRIFT

(11) **EP 1 898 779 B1**
(45) Veröffentlichungstag und Bekanntmachung des Hinweises auf die Patenterteilung: **09.09.2009**
(21) Anmeldenummer: 06828784.6
(22) Anmeldetag: 17.05.2006
(51) Int. Cl.: A61B 5/00, G01N 21/64

(54) **SONDE ZUR MESSUNG DES SAUERSTOFFGEHALTES IN BIOLOGISCHEM GEWEBE SOWIE KATHETER MIT EINER DERARTIGEN SONDE**
PROBE FOR MEASURING THE OXYGEN CONTENT IN BIOLOGICAL TISSUE, AND CATHETER WITH SUCH A PROBE
SONDE SERVANT A MESURER LA TENEUR EN OXYGENE D'UN TISSU BIOLOGIQUE ET CATHETER POURVU D'UNE SONDE DE CE TYPE

(30) Priorität: 25.05.2005 DE 102005024578
(43) Veröffentlichungstag der Anmeldung: 19.03.2008
(73) Patentinhaber: RAUMEDIC AG, 95233 Helmbrechts (DE)
(72) Erfinder: KUNZE, Gerd, 08297 Zwönitz (DE)
(74) Vertreter: Hofmann, Matthias
(86) Internationale Anmeldenummer: PCT/EP2006/004664
(87) Internationale Veröffentlichungsnummer: WO 2007/033707

(56) Entgegenhaltungen:
- EP-A- 0 503 812
- US-A- 4 476 870
- US-A- 4 737 343

## Beschreibung

Die Erfindung betrifft eine Sonde zur Messung des Sauerstoffgehaltes in biologischem Material nach dem Oberbegriff des Anspruchs 1 sowie nach dem Oberbegriff des Anspruchs 9. Ferner betrifft die Erfindung einen Katheter mit einer derartigen Sonde.

Die Messung von Sauerstoff ist insbesondere im Bereich der Medizin von großem Interesse. Hierbei ist insbesondere die in vivo-Erfassung des gelösten, nicht an Hämoglobin gebundenen Sauerstoffs für die Beurteilung der Versorgung des biologischen Materials, insbesondere des Gewebes, von Bedeutung. Weitere Beispiele für hinsichtlich des Sauerstoffgehaltes zu untersuchendes biologisches Material sind Körperflüssigkeiten wie Blut oder Liquor. Eine entscheidende Größe ist dabei der Sauerstoffpartialdruck, der im untersuchten Gewebe herrscht. Der Partialdruck des in der interstitiellen Flüssigkeit physikalisch gelösten Sauerstoffes entspricht der Verfügbarkeit von Sauerstoff auf zellulärer Ebene. Insbesondere im Bereich der kardiovaskulären sowie der neurochirurgischen Anwendung als auch im Bereich der Transplantationsmedizin findet die Erfassung des Gewebesauerstoffs Anwendung. Hierbei werden zur Messung vor allem Katheter eingesetzt, die spezifisch auf Sauerstoff reagierende Sensorsysteme bzw. Sonden umfassen.

Eine Sonde der eingangs genannten Art ist bekannt aus der WO 93/05702 A1. Weitere Sonden, die faseroptisch Sauerstoffparameter von Gewebe messen, sind bekannt aus der US 5,673,694, der US 5,995,208, der US 5,579,774 sowie den dort zitierten Veröffentlichungen. Eine weitere faseroptische Sauerstoffsonde ist bekannt aus J.I. Peterson et al., Anal. Chem. 1984, 56, 62-67. Weitere faseroptische Sonden sind bekannt aus der US 4,752,115 A, US 5,142,155 A und US 4,861,727 A.

Eine Sonde gemäß Oberbegriff der Ansprüche 1 und 9 ist bekannt aus US-A-47 37 343.

Eine bekannte Messtechnik zur faseroptischen Messung des Partialdrucks des physikalisch gelösten, also freien Sauerstoffs, ist das dynamische Sauerstoffquenchen. Hierbei wird ein in einer Matrix eingebetteter Fluoreszenzfarbstoff, zum Beispiel ein Platinkomplex, am distalen Ende der optischen Faser angebracht. Der Fluoreszenzfarbstoff wird über die Faser optisch angeregt, meist durch Laserstrahlung, welche auf Absorptionsbanden des Farbstoffs abgestimmt ist. Die so angeregten Farbstoffmoleküle gehen mit zeitlicher Verzögerung, z.B. im Bereich zwischen 1 und 60 µs, unter Aussenden Lichtes gleicher oder rotverschobener Wellenlänge in den Grundzustand über. In Gegenwart von Sauerstoff kann dieser Übergang durch Stoßprozesse auch strahlungslos in den Grundzustand erfolgen.

Hierdurch nimmt die Intensität des durch die Faser zurückgestrahlten Lichtes ab. Diese Reduktion ist dem freien Sauerstoff in der direkten Umgebung um den Fluoreszenzfarbstoff proportional. Die bekannten faseroptischen Sensoren sind gegenüber Streulicht und intensitätsbeeinflussenden Faktoren wie Haarrissen oder einer Faserkrümmung äußerst sensibel. Diese Sensibilität kann verringert werden, wenn mit Hilfe einer Lock-In-Technik die Phasenverschiebung des vom Fluoreszenzfarbstoff zurückgestrahlten Lichts gegenüber dem eingestrahlten Licht gemessen wird. Hierbei wird ausgenutzt, dass langlebige Fluoreszenzzustände statistisch anfälliger für die strahlungslosen Stoßprozesse des dynamischen Sauerstoffquenchens sind. Eine, wenn auch verringerte Sensibilität bekannter faseroptischer Sensoren gegenüber Streulicht und intensitätsbeeinflussenden Faktoren liegt allerdings auch dann vor, wenn mit Lock-In-Technik gemessen wird. Zudem hat sich herausgestellt, dass in derselben Geweberegion mit den bekannten faseroptischen Sensoren sehr unterschiedliche Messwerte des freien Sauerstoffgehaltes resultieren, was die Interpretation einer Einzelmessung nahezu unmöglich macht.

Es ist daher eine Aufgabe der vorliegenden Erfindung, eine Sonde der eingangs genannten Art derart weiterzubilden, dass die Sensibilität der Faser am Messort gegenüber störenden Umgebungseinflüssen verringert ist und die Interpretationsmöglichkeiten der Messergebnisse verbessert sind.

Diese Aufgabe ist erfindungsgemäß gelöst durch eine Sonde mit den im Kennzeichnungsteil des Anspruchs 1 und 9 angegebenen Merkmalen.

Erfindungsgemäß wurde erkannt, dass die Schaffung eines den distalen Faserabschnitt umgebenden Gasraumes durch eine sauerstoffdurchlässige und gleichzeitig flüssigkeitsundurchlässige Membran das Messvolumen um den Farbstoff herum vorteilhaft vergrößert. Das Messvolumen ist nun nicht mehr reduziert auf die unmittelbare Material- bzw. Gewebeumgebung um den Farbstoff herum, sondern erweitert auf die äußere Umgebung der den Gasraum vorgebenden Membran. Der im Gasraum sich bildende Sauerstoff-Partialdruck ist somit ein Maß für das Mittel des freien Sauerstoffgehaltes an der Außenfläche der den Gasraum vorgebenden Membran. Die Vergrößerung des sensitiven Volumens führt damit zu einer medizinisch gut verwertbaren Aussage über die lokale, nicht aber punktuelle Sauerstoffversorgung im die Sonde umgebenden biologischen Gewebe. Damit kann der Gewebezustand besser beurteilt werden als bei der rein punktuellen Messung, die bei den faseroptischen Messverfahren des Standes der Technik gegeben ist. Gleichzeitig schützt die Membran den distalen Faserabschnitt im Gasraum, sodass dort die Gefahr einer Störung der Messung verhindert ist. Die Robustheit des erfindungsgemäßen faseroptischen Sensors wird durch Einsatz der vorstehend erwähnten Lock-In-Technik noch weiter erhöht. Mit dem faseroptischen Sensor kann der Sauerstoffgehalt von Gewebe, aber auch von anderem biologischen Material, zum Beispiel von Körperflüssigkeiten wie Blut oder Liquor, vermessen werden. Beim sauerstoffsensitiven Farbstoff kann es sich zum Beispiel um einen Platinkomplex oder um einen Rutheniumkomplex handeln. Der sauerstoffsensitive Farbstoff liegt als Beschichtung vor oder ist zumindest abschnittsweise in die Membranwand eingearbeitet. Die Anordnung des Farbstoffes muss dabei natürlich so sein, dass ein möglichst direkter optischer Weg zwischen den Farbstoffmolekülen und der distalen Endfläche der Faser vorhanden ist. Vorzugsweise ist daher der Farbstoff als direkte Beschichtung auf die distale Endfläche der Faser vorgesehen. Gegenüber einer vollständigen Füllung eines der distalen Faser-Endfläche vorgelagerten Volumens mit Farbstoff hat die erfindungsgemäße Anordnung des Farbstoffs als Beschichtung oder Membranwandbestandteil den Vorteil, dass eine Lichtantwort des Farbstoffs nicht durch andere im Volumen enthaltene Farbstoffmoleküle unerwünscht absorbiert wird und damit verloren geht.

Eine einheitliche Membranstärke nach Anspruch 2 verhindert eine zeitliche Verschmierung des Partialdruck-Messsignales, da die freien Sauerstoffmoleküle eine einheitliche Diffusionszeit durch die Membran haben. Es resultiert eine homogene Sensorcharakteristik. Eine einheitliche Membranstärke bedeutet nicht, dass die Membranstärke über die gesamte Fläche der Membran exakt konstant ist. Geringfügige Abweichungen von einer mittleren Membranstärke, welche die angesprochene homogene Sensorcharakteristik in der Praxis nicht beeinflussen, können hingenommen werden. Derartige tolerable Abweichungen bewegen sich beispielsweise im Bereich von 200 µm. Ein sauerstoffsensitiver Farbstoff mit einer langen Fluoreszenzlebensdauer kann störende Effekte, die von Abweichungen der Membranstärke herrühren, ausgleichen. Daher ist für eine homogene Sensorcharakteristik zum Beispiel ein Platinkomplex mit einer Fluoreszenzlebensdauer bis zu 60 µs von Vorteil.

Ein Gasraum nach Anspruch 3 lässt sich mit einer kostengünstig herstellbaren Membran realisieren. Wenn die Gasraum-Längsachse parallel zur Faserachse und von dieser beabstandet ist, kann der Gasraum mit einem großen zusammenhängenden freien Volumen gestaltet werden, welches sich zur Anordnung weiterer Komponenten, insbesondere von Sensoren, der Sonde eignet. Wenn die Achsen zusammenfallen, resultiert ein rotationssymmetrischer Aufbau, der insbesondere Herstellungsvorteile bietet. Bei zusammenfallenden Achsen bietet sich insbesondere eine Ausgestaltung an, bei der die distale Endfläche der Faser mit dem Farbstoff im Gasraum zentriert vorliegt, so dass im Bezug auf freien Sauerstoff, der durch die Membran diffundiert, eine Diffusionslängensymmetrie vorliegt, was die Messqualität erhöhen kann.

Eine Membran nach Anspruch 4 lässt sich einfach herstellen, da der Membranschlauch z. B. durch Ablängen eines Endlosschlauchs gebildet werden kann.

Materialien nach Anspruch 5 haben sich hinsichtlich der Eigenschaften Sauerstoffdurchlässigkeit und Flüssigkeitsundurchlässigkeit als für die Anwendung in der Sonde gut geeignet herausgestellt.

Eine Membran nach Anspruch 6 passt sich gut an das sie umgebende Gewebe an, sodass eine Verfälschung der Messung verhindert ist.

Eine Luftfüllung nach Anspruch 7 verhindert, dass sich die Gaszusammensetzung bei einer Lagerung der Sonde vor deren Einsatz verändert. Altemativ ist es möglich, ein Gas oder ein Gasgemisch vor Einsatz der Sonde einzufüllen, welches aus derart großen Molekülen besteht, dass diese die sauerstoffdurchlässige Membran nicht nach außen durchdringen können. Auch in diesem Fall wird eine Gasraumfüllung für eine Lagerung der Sonde vor deren Einsatz erzielt, die sich nicht verändert.

Eine Wasserdampfdurchlässigkeit nach Anspruch 8 ermöglicht aufgrund der erhöhten Wärmekapazität des Gases im Gasraum durch Wasserdampf, der dort eindringen kann, eine schnellere Anpassung der sich im Gasraum befindlichen Sensoren an die Umgebungstemperatur. Hierdurch ist innerhalb des Gasraums eine verlässliche Temperaturmessung möglich, ohne dass lange auf die Einpendelung eines thermischen Gleichgewichts gewartet werden muss. Wird daher auf eine hohe Wasserdampfdurchlässigkeit Wert gelegt, kann die Membran insbesondere aus Tetrafluorethylen-Hexafluorpropylen-Copolymer (FEP) ausgeführt sein. Auch eine Membran aus Polyethylen (PE) weist eine, im Vergleich zu FEP allerdings geringere, Wasserdampfdurchlässigkeit auf.

Die Sonde nach Anspruch 9 kann diejenigen Merkmale aufweisen, die vorstehend im Zusammenhang mit den Ansprüchen 2 bis 8 erläutert wurden.

Eine weitere Aufgabe ist es, einen Katheter anzugeben, mit dem eine aussagekräftige Messung durch eine erfindungsgemäße Sonde ermöglicht ist.

Diese Aufgabe ist erfindungsgemäß gelöst durch einen Katheter mit den im Anspruch 10 angegebenen Merkmalen.

Der Temperatursensor erlaubt eine Kompensation einer thermischen Abhängigkeit der Sauerstoffgehaltsmessung. Der vorzugsweise vorhandene Drucksensor ermöglicht eine zusätzliche Druckmessung, die in Kombination mit der Sauerstoffgehaltsmessung wertvolle gewebespezifische Informationen liefert. Durch eine derartige Kombinationsmessung, bei der Sauerstoffgehalt und Druck gemessen werden, lässt sich zum Beispiel prüfen, inwieweit der Sauerstoffgehalt und der Druckverlauf in ihrer Dynamik korreliert sind. Es kann also eine Korrelation des Gewebedrucks und des Sauerstoffpartialdrucks ermittelt werden. Das Erfassen verschiedener physiologischer Parameter über einen einzigen Katheter reduziert das Infektions-und Blutungsrisiko im Vergleich zur Applikation mehrerer einzelner Katheter über jeweils getrennte Katheterzugänge. Die vorzugsweise teilweise metallische Katheterspitze ermöglicht deren Erkennen in bildgebenden Verfahren, zum Beispiel bei der CT. Dadurch wird die gezielte Platzierung in das gewünschte Areal ermöglicht. Dies ist insbesondere notwendig zur Differenzierung zwischen einer lokalen oder globalen Situation bei pathophysiologischen Ereignissen mit erniedrigten Sauerstoffpartialdruckwerten, wie zum Beispiel bei Blutungen im Stichkanal, Schwellung im Bereich der Katheterlage oder bei einer lokalen Ischämie. Weitere Vorteile des Katheters sind diejenigen, die oben im Zusammenhang mit der Sonde schon erwähnt wurden.

Ein Temperatursensor nach Anspruch 11 erlaubt eine gute Kompensation der Temperaturabhängigkeit der faseroptischen Sauerstoffgehaltsmessung, da die Temperatur genau dort gemessen wird, wo auch die Sauerstoffgehaltsmessung stattfindet. Durch die permanente Temperaturkorrektur der Sauerstoffgehaltsmessung sind die Werte auch bei Hypo- und Hyperthermie verlässlich.

Eine Katheterspitze nach Anspruch 12 führt zu einer Reduktion der Einzelteile des Katheters.

Ausführungsbeispiele der Erfindung werden nachfolgend anhand der Zeichnung näher erläutert. In dieser zeigen:
- Fig. 1: im schematischen Längsschnitt eine Sonde zur Messung des Sauerstoffgehaltes in biologischem Gewebe;
- Fig. 2: eine zu Fig. 1 ähnliche Darstellung der Sonde, bei der ein distaler Faserabschnitt einer optischen Faser weiter in einen durch eine Membran vorgegebenen Gasraum eingeschoben ist;
- Fig. 3: einen Schnitt gemäß Linie III-III in Fig. 2;
- Fig. 4: einen zu Fig. 3 ähnlichen Schnitt durch eine weitere Ausführungsform einer Sonde;
- Fig. 5: einen Längsschnitt durch einen Katheter mit einer weiteren Ausführung einer Sonde zur Messung des Sauerstoffgehalts in biologischem Gewebe;
- Fig. 6: schematisch einen Schnitt gemäß Linie VI-VI in Fig. 5;
- Fig. 7: einen zu Fig. 5 ähnlichen Schnitt durch eine weitere Ausführungsform eines Katheters; und
- Fig. 8: eine Stirnansicht gemäß Sichtpfeil VIII in Fig. 7.

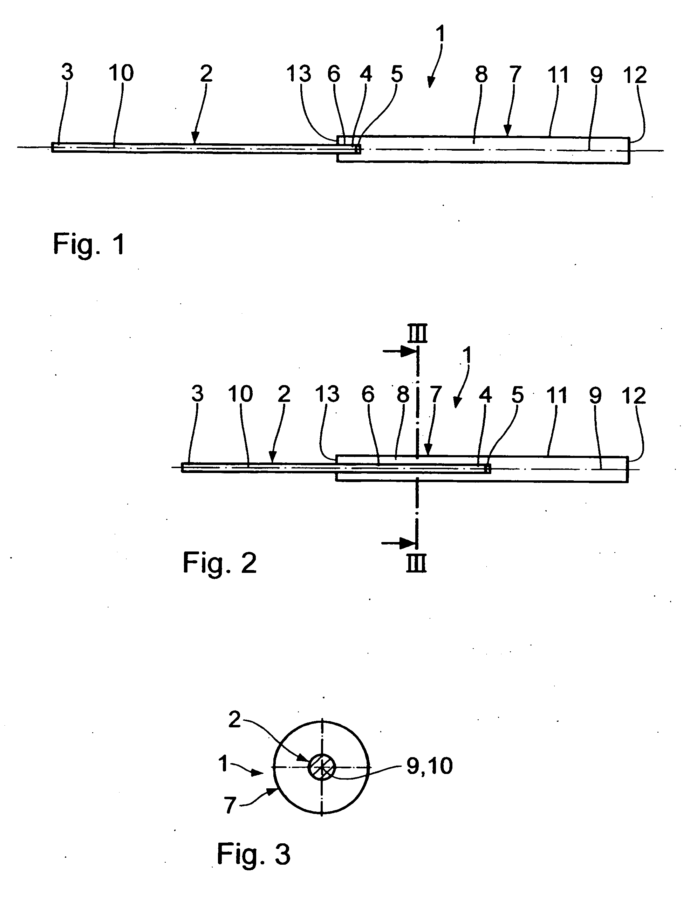

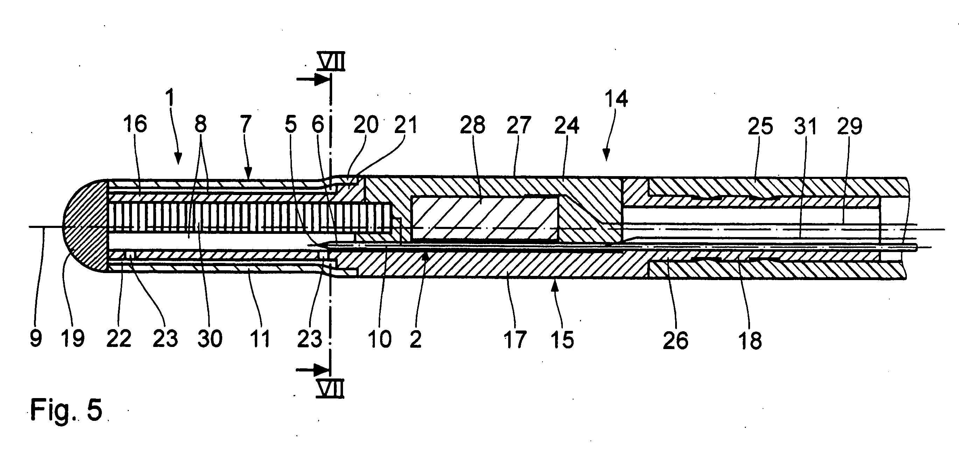

Die Fig. 1 bis 3 zeigen eine erste Ausführungsform einer Sonde zur Messung des Sauerstoffgehalts in biologischem Gewebe. Die Sonde 1 kann Bestandteil eines Katheters z. B. nach Art desjenigen sein, der in Fig. 5 dargestellt ist.

Die Sonde 1 weist eine optische Faser 2 auf. Ein proximales Ende 3, welches dem zu vermessenden biologischen Gewebe abgewandt ist, ist an eine Lichtquelle einerseits und an einen Lichtsensor andererseits optisch ankoppelbar. Bei der optischen Faser 2 kann es sich um eine Einzelfaser oder um ein Faserbündel handeln.

An einer distalen Endfläche 4 der optischen Faser 2, die dem zu vermessenden biologischen Gewebe zugewandt ist, ist ein sauerstoffsensitiver Farbstoff 5 angeordnet. Der Farbstoff 5 ist an die distale Endfläche 4 der optischen Faser 2 optisch angekoppelt. Die distale Endfläche 4 ist mit dem Farbstoff 5 beschichtet. Ein distaler Faserabschnitt 6 einschließlich der distalen Endfläche 4 mitsamt dem Farbstoff 5 ist umschlossen von einer sauerstoffdurchlässigen, flüssigkeitsundurchlässigen Membran 7. Die Membran 7 ist wasserdampfdurchlässig ausgeführt. Letztere gibt im umschlossenen Bereich einen Gasraum 8 vor, der die distale Endfläche 4 mit dem Farbstoff 5 umgibt. Alternativ zu einer Beschichtung der distalen Endfläche 4 mit dem Farbstoff 5 ist es möglich, die Innenwand der Membran 7 zumindest bereichsweise mit dem Farbstoff 5 zu beschichten. Als beschichtete Bereiche werden dabei diejenigen ausgewählt, die von der distalen Endfläche 4 "gesehen" werden, zu denen also ein direkter optischer Weg von der distalen Endfläche 4 aus vorhanden ist. Bei einer weiteren Variante ist es möglich, den Farbstoff 5 in die Wandung der Membran 7 einzuarbeiten.

Die Membran 7 hat dort, wo sie den Gasraum 8 vorgibt, eine einheitliche Stärke. Die zulässige Abweichung der Membranstärke von einem Vorgabewert hängt von der gewünschten Messdynamik des Sauerstoffpartialdruckes ab. Für Messungen im Hirngewebe haben sich zum Beispiel Abweichungen von 200 µm als tolerierbar herausgestellt. Bei der Sonde nach den Fig. 1 bis 3 hat der Gasraum 8 die Gestalt eines Zylinders. Eine Gasraum-Längsachse 9 fällt mit einer Faserachse 10 zumindest im distalen Faserabschnitt 6 zusammen.

Bei der Ausführung nach den Fig. 1 bis 3 ist die Membran 7 aus Silikonkautschuk. Alternativ kann die Membran 7 auch aus einem der Materialien Polyethylen (PE), Teflon (PTFE) oder Tetrafluorethylen-Hexafluorpropylen-Copolymer (FEP) sein. Die Membran 7 ist derart flexibel, dass sie unter Einfluss eines im Gasraum 8 vorliegenden Gasdrucks verformbar ist. Die Gestalt des Gasraums 8 kann sich also an den jeweils dort vorliegenden Gasdruck in Abhängigkeit vom Umgebungsdruck anpassen.

Je nach Anwendung kann die Sonde 1 mit unterschiedlichen Relativpositionen der Faser 2 zur Membran 7 vorliegen. In der Position nach Fig. 1 ist die Faser 2 lediglich ein kurzes Stück weit in den Gasraum 8 eingeschoben, sodass der von der Membran 7 umschlossene distale Faserabschnitt 6 im Vergleich zur Länge des Gasraums 8 kurz ist. In der Position nach Fig. 2 ist die Faser 2 weiter in den Gasraum 8 eingeschoben, sodass der distale Faserabschnitt 6 in etwa halb so lang ist wie der Gasraum 8. In der Position nach Figur 2 liegt die distale Endfläche 4 mit der Faser 5 symmetrisch zentral im Gasraum 8, so dass in Bezug auf freien Sauerstoff, der durch die Membran 7 diffundiert, eine Diffusionslängensymmetrie vorliegt.

### Die Sonde 1 wird folgendermaßen eingesetzt:

Zunächst wird die Sonde 1, gegebenenfalls mit samt einem diese aufweisenden Katheter, in vivo beim Patienten in Messposition gebracht. Der Gasraum 8 ist vor dem Einsatz der Sonde 1 mit Luft gefüllt. Sowohl die Lichtquelle als auch der Lichtsensor werden an die Faser 2 proximal angekoppelt. Die Membran 7 ist nach außen hin vom biologischen Gewebe des Patienten umschlossen. Freier, das heißt nicht an Hämoglobin gebundener Sauerstoff, kann die Membran 7 von außen her durchdringen, also in den Gasraum 8 eindringen. Da der Gasraum 8 flüssigkeitsdicht nach außen abgeschlossen ist, kann weder Flüssigkeit noch Gewebe in den Gasraum 8 eindringen.

Der Farbstoff 5 ist auf die Wellenlänge des eingekoppelten Lichtes so abgestimmt, dass als Folge des in den Farbstoff 5 eingekoppelten Lichtes unter Einfluss der im Gasraum 8 vorliegenden Sauerstoffmoleküle vom Farbstoff 5 in die optische Faser 2 zurückgekoppeltes Licht in seiner vom Lichtsensor messbaren Menge von der Konzentration des freien Sauerstoffs im Gasraum 8 abhängt. Durch die einheitliche Stärke der den Gasraum 8 vorgebenden Membran 7 ist entsprechend eine einheitliche Eindringzeit des freien Sauerstoffs vom die Membran 7 umgebenden biologischen Gewebe in den Gasraum 8 gewährleistet. Messfehler aufgrund unterschiedlicher Eindringzeiten können daher nicht entstehen.

Mithilfe des Lichtsensors wird die Menge des vom Farbstoff 5 in die Faser 2 zurückgekoppelten Lichtes als Antwort auf das von der Lichtquelle in die Faser 2 eingekoppelte Licht gemessen. Diese zurückgekoppelte Lichtmenge ist ein Maß für den Sauerstoffgehalt im Gasraum 8 und damit ein Maß für den im die Membran 7 umgebenden biologischen Gewebe nicht an Hämoglobin gebundenen, also freien Sauerstoff. Alternativ ist es möglich, die Phasenverschiebung des zurückgekoppelten Lichtes in Abhängigkeit von der Phase des eingekoppelten Lichtes zum Beispiel mit Hilfe von Lock-In-Technik zu messen. Da langlebige Zustände des Farbstoffs 5 statistisch anfälliger für einen sauerstoffinduzierten strahlungslosen Übergang in den Grundzustand durch einen Stoßprozess sind, verschiebt sich die mittlere Lebensdauer der Fluoreszenzzustände, die zum zurückgekoppelten Licht beitragen, was wiederum zu einer messbaren Phasenverschiebung gegenüber dem eingestrahlten Signal, welches als Lock-In-Referenz genutzt werden kann, führt.

Bei der Ausführung nach den Fig. 1 bis 3 ist die Membran 7 einstückig ausgeführt. Das Material der Membran 7 dichtet im Bereich des Fasereintritts in den Gasraum 8 gegen die optische Faser 2 ab.

Bei einer Variante der Sonde 1, die nachfolgend der Einfachheit halber ebenfalls anhand der Fig. 1 bis 3 beschrieben wird, umfasst die Membran 7 einen Membranschlauch 11, der die Mantelwand des zylindrischen Gasraums 8 vorgibt. Ein faserabgewandtes, stirnseitiges Ende des Membranschlauchs 11 weist einen Dichtungsdeckel 12 auf. Der Dichtungsdeckel 12 kann aus dem gleichen Material wie der Membranschlauch 11 sein. Alternativ ist es möglich, den Dichtungsdeckel 12 aus einem anderen, insbesondere vollkommen fluidundurchlässigen, Material als den Membranschlauch 11 herzustellen, da es ausreicht, wenn der Membranschlauch 11 sauerstoffdurchlässig ist. An der der Faser zugewandten Seite ist der Membranschlauch 11 gegen die Faser 2 mittels eines Dichtungsrings 13 abgedichtet, der aus dem gleichen Material sein kann wie der Dichtungsdeckel 12.

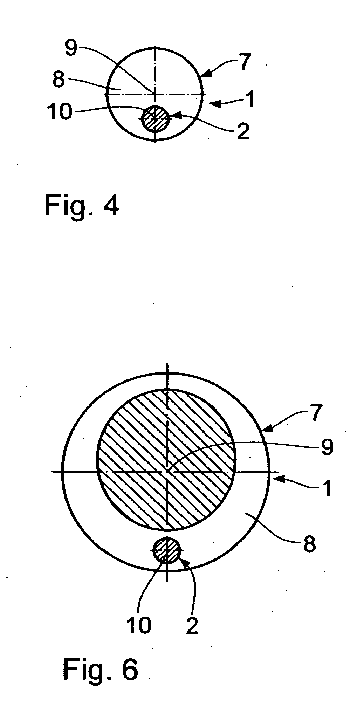

Die Ausführung der Sonde 1 nach Fig. 4 unterscheidet sich von derjenigen nach den Fig. 1 bis 3 lediglich dadurch, dass bei der Sonde nach Fig. 4 die Gasraum-Längsachse 9 nicht mit der Faserachse 10 im Gasraum 8 zusammenfällt, sondern parallel zu dieser ist. Dadurch weist der Gasraum 8 bei der Ausführung nach Fig. 4 ein größeres zusammenhängendes freies Volumen auf, in dem weitere Komponenten, z.B. weitere Sensoren, untergebracht werden können.

Die Fig. 5 und 6 zeigen einen Katheter 14 mit einer weiteren Ausführung einer Sonde 1. Der Katheter 14 wird nachfolgend nur dort beschrieben, wo er sich von dem unterscheidet, was vorstehend im Zusammenhang mit den Fig. 1 bis 4 schon ausgeführt wurde. Komponenten, die denjenigen entsprechen, die vorstehend unter Bezugnahme auf die Fig. 1 bis 4 schon beschrieben wurden, tragen die gleichen Bezugsziffern und werden nur dort beschrieben, wo sie sich in Aufbau und Funktion von den Komponenten nach den Fig. 1 bis 4 unterscheiden. Der Katheter 14 hat ein Gehäuse 15.. Dieses ist bei der dargestellten Ausführung aus Titan, kann aber auch aus einem anderen Werkstoff gefertigt sein. Das Gehäuse 15 ist einstückig und strukturell unterteilt in einen distalen Gehäuseabschnitt 16, einen Gehäuse-Mittelabschnitt 17 und einen proximalen Gehäuseabschnitt 18. An seinem distalen Ende wird der distale Gehäuseabschnitt 16 abgedeckt von einer atraumatischen Katheterspitze 19. Die Katheterspitze 19 geht umfangseitig des distalen Gehäuseabschnitts 16 über in den Membranschlauch 11 der Membran 7.

Die Katheterspitze 19 stellt einen Dichtungsdeckel der Membran 7 dar. Ein proximaler Umfangs-Endabschnitt 20 der Membran 7 ist auf eine Umfangsstufe 21 des Gehäuse-Mittelabschnitts 17 aufgeschoben. Der Außendurchmesser der Umfangsstufe 21 ist etwas größer als der Innendurchmesser des Membranschlauchs 11.

Zwischen dem Membranschlauch 11 und dem distalen Gehäuseabschnitt 16 liegt ein Ringraum 22 vor, der Teil des Gasraums 8 ist und über Durchbrechungen 23 mit einem zylindrischen Innenraum im distalen Gehäuseabschnitt 16, der ebenfalls Teil des Gasraums 8 ist, in Verbindung steht. In diesen Innenraum ist der distale Faserabschnitt 6 der optischen Faser 2 mit dem Farbstoff 5 eingeschoben. Im weiteren Verlauf durchtritt die Faser 2 zunächst einen Dichtkörper 24, der in den Gehäuse-Mittelabschnitt 17 eingesetzt ist und zum Beispiel aus Silikonkautschuk gefertigt sein kann. Im weiteren Verlauf durchtritt die Faser 2 einen zylindrischen Innenraum des proximalen Gehäuseabschnitts 18 sowie einen Katheterschlauch 25. Letzterer ist auf eine Umfangsstufe 26, die im proximalen Gehäuseabschnitt 18 ausgebildet ist, aufgeschoben.

Eine Außenwand 27 des Dichtkörpers 24 ist in einem Gehäusefenster im Gehäuse-Mittelabschnitt 17 angeordnet und fluchtet mit der sie umgebenden Außenwand des Gehäuse-Mittelabschnitts 17. Im Dichtkörper 24 ist ein Drucksensor 28 angeordnet. Über eine Signalleitung 29, die durch den Dichtkörper 24, den proximalen Gehäuseabschnitt 18 sowie den Katheterschlauch 25 verläuft, steht der Drucksensor 28 mit einer nicht dargestellten zentralen Steuer- und Auswerteeinheit in Verbindung.

Wie bei der Ausführung nach Fig. 4 fällt bei der Sonde 1 nach Fig. 5 und 6 die Gasraum-Längsachse 9 nicht mit der Faserachse 10 zusammen, sodass im vom distalen Gehäuseabschnitt vorgegebenen Innenraum ein großes zusammenhängendes freies Volumen vorliegt. Dort ist im Innenraum ein Temperatursensor 30 angeordnet. Ein proximales Ende des Temperatursensors 30 ist dicht in den Dichtkörper 24 eingesetzt. Über eine Signalleitung 31 steht der Temperatursensor 30 mit der zentralen Steuer- und Auswerteeinheit in Verbindung. Die Signalleitung 31 durchläuft ebenfalls den Dichtkörper 24, den proximalen Gehäuseabschnitt 18 sowie den Katheterschlauch 25.

Die Funktion des Katheters 14 wird nachfolgend nur dort beschrieben, wo ein Unterschied zum Einsatz der Sonde 1 nach den Fig. 1 bis 4 vorliegt. Nachdem der Katheter 14 in die Messposition im Patienten gebracht ist, wird mit der Sonde 1 entsprechend dem, was oben im Zusammenhang mit der Ausführung nach den Fig. 1 bis 4 ausgeführt wurde, der Sauerstoffgehalt des den Katheter 14 umgebenden biologischen Gewebes gemessen. Gleichzeitig wird über den Drucksensor 28 der Druck, den das biologische Gewebe über die Außenwand 27 auf den Drucksensor 28 ausübt, sowie die Temperatur im Gasraum 8 über den Temperatursensor 30 gemessen. Die Messwerte werden über die Signalleitungen 29 und 31 an die zentrale Steuer- und Auswerteeinheit, an die auch die Lichtquelle und der Lichtsensor der Sonde 1 angeschlossen sind, zugeleitet. Nach Einstellung des thermischen Gleichgewichts entspricht die Temperatur im Gasraum 8, die der Temperatursensor 30 misst, der Temperatur des den distalen Gehäuseabschnitt 16 des Katheters 14 umgebenden biologischen Gewebes. Diesen Temperaturausgleich bewerkstelligt und die Grundlage für die schnelle Temperaturmessung ist Wasserdampf, welcher den Membranschlauch 11 durchdringt und in dem Gasraum 8 eindringt. Aufgrund der über den Temperatursensor 30 gemessenen Temperatur kann die Temperaturabhängigkeit des Wasserdampfs-Partialdrucks bei der Sauerstoff-Partialdruckmessung über die optische Faser 2 berücksichtigt werden.

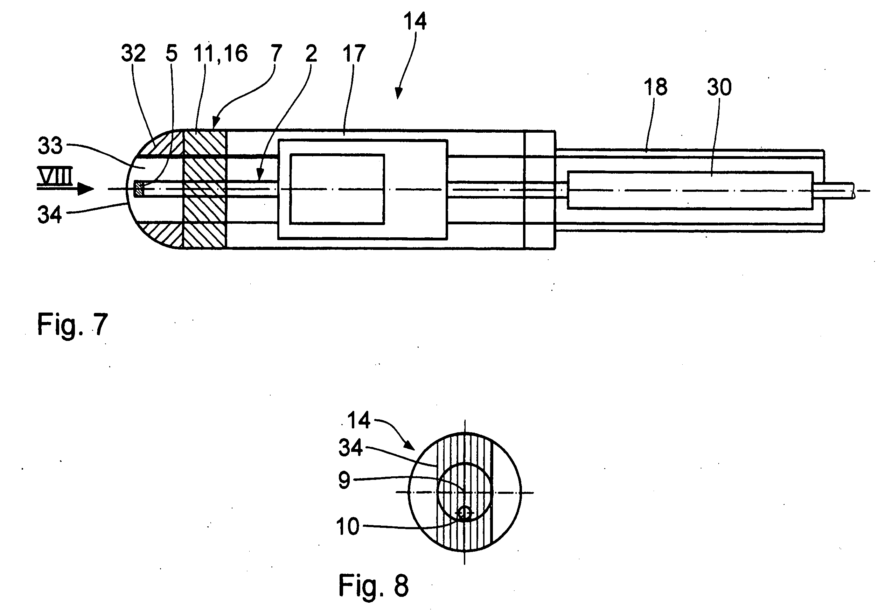

Die Fig. 7 und 8 zeigen eine weitere Ausführung eines Katheters mit einer Sonde zur Messung des Sauerstoffgehaltes in biologischem Gewebe. Komponenten, die vorstehend schon unter Bezugnahme auf die Fig. 1 bis 6 beschrieben wurden, tragen die gleichen Bezugsziffern und werden nicht nochmals im Einzelnen erläutert.

Der Katheter 14 nach den Fig. 7 und 8 unterscheidet sich von dem nach den Fig. 5 und 6 hauptsächlich durch die Gestaltung der Membran 7 und die Anordnung der Sensoren. Eine Katheterspitze 32 ist bei der Ausführung nach den Fig. 7 und 8 nicht wie bei der Ausführung nach den Fig. 5 und 6 aus Vollmaterial ausgeführt, sondern weist eine innere Ausnehmung 33 auf, die Teil des Gasraums 8 ist. Distal wird die Ausnehmung 33 abgedeckt von einem stirnseitigen Membranabschnitt 34, der aus dem gleichen Material gefertigt ist und die gleiche Materialstärke aufweist wie der Membranschlauch 11. Der Membranabschnitt 34 geht randseitig nahtlos in die diesen umgebenden Abschnitte der Katheterspitze 32 über, sodass der Membranabschnitt 34 zusammen mit diesen umgebenden Abschnitten die atraumatische Katheterspitze bildet. Der Membranabschnitt 34 ist in der Stirnansicht der Figur 8 durch eine parallele Schraffur angedeutet. Die optische Faser 2 mit dem Farbstoff 5 ist bei der Ausführung nach den Fig. 7 und 8 bis in die Ausnehmung 33 der Katheterspitze 32 eingeschoben.

Auch bei der Ausführung nach den Fig. 7 und 8 fällt die Gasraum-Längsachse 9 nicht mit der Faserachse 10 zusammen, sondern ist zu dieser beabstandet und parallel.

Der Temperatursensor 30 ist bei der Ausführung nach den Fig. 7 und 8 nicht im Gasraum 8, sondern im proximalen Gehäuseabschnitt 18 angeordnet.

Die Funktion des Katheters 14 nach den Fig. 7 und 8 entspricht derjenigen des Katheters nach den Fig. 5 und 6. Beim Katheter 14 nach den Fig. 7 und 8 wird die Temperatur im Bereich des proximalen Gehäuseabschnitts 18 gemessen, sodass eine korrekte Berücksichtigung der Temperaturabhängigkeit des Wasserdampf-Partialdrucks voraussetzt, dass die Temperatur des biologischen Gewebes im Bereich des proximalen Gehäuseabschnitts 18 mit der Temperatur im Bereich des distalen Gehäuseabschnitts 16 übereinstimmt.

Als Farbstoff 5 können Platin- oder Rutheniumkomplexe eingesetzt werden. Typische Fluoreszenzlebensdauern von Platinkomplexen sind 60µs bei 0% Luftsättigung und 20 µs bei 100% Luftsättigung. Typische Fluoreszenzlebensdauem von Rutheniumkomplexen sind etwa 6 µs bei 0% Luftsättigung und etwa 4 µs bei 100% Luftsättigung.

## Patentansprüche

1. Sonde (1) zur Messung des Sauerstoffgehaltes in biologischem Material
- mit mindestens einer optischen Faser (2), welche proximal an eine Lichtquelle einerseits und an einen Lichtsensor andererseits optisch ankoppelbar ist,
- mit einem sauerstoffsensitiven Farbstoff (5), welcher an einer distalen Endfläche (4) der Faser (2) angeordnet und an diese optisch angekoppelt ist,
**dadurch gekennzeichnet, dass** ein distaler Faserabschnitt (6) einschließlich der distalen Endfläche (4) mitsamt dem Farbstoff (5) umschlossen ist von einer sauerstoffdurchlässigen, flüssigkeitsundurchlässigen Membran (7), die im umschlossenen Bereich einen die distale Endfläche (4) mit dem Farbstoff (5) umgebenden Gasraum (8) vorgibt, wobei der Farbstoff (5)
- als Beschichtung auf der distalen Endfläche (4) vorgesehen ist.

2. Sonde nach Anspruch 1, **dadurch gekennzeichnet, dass** die Stärke der Membran (7) dort, wo sie den Gasraum (8) vorgibt, einheitlich ist.

3. Sonde nach Anspruch 1 oder 2, **dadurch gekennzeichnet, dass** der Gasraum (8) zumindest abschnittsweise die Gestalt eines Zylinders hat, dessen Längsachse (9) parallel zur Faserachse (10) im distalen Faserabschnitt (6) ist oder mit ihr zusammenfällt.

4. Sonde nach einem der Ansprüche 1 bis 3, **dadurch gekennzeichnet, dass** die Membran (7) einen Membranschlauch (11) umfasst, dessen Enden (12, 13) zur Vorgabe des Gasraums (8) gegen Flüssigkeitsdurchtritt abgedichtet sind.

5. Sonde nach einem der Ansprüche bis 4, **dadurch gekennzeichnet, dass** die Membran (7) aus einem der Materialien Silikonkautschuk, PE, PTFE oder FEP gebildet ist.

6. Sonde nach einem der Ansprüche 1 bis 5, **dadurch gekennzeichnet, dass** die Membran (7) derart flexibel ist, dass sie unter Einfluss eines im Gasraum (8) vorliegenden Gasdrucks verformbar ist.

7. Sonde nach einem der Ansprüche 1 bis 6, **dadurch gekennzeichnet, dass** der Gasraum (8) vor Einsatz der Sonde (1) mit Luft gefüllt ist.

8. Sonde nach einem der Ansprüche 1 bis 7, **dadurch gekennzeichnet, dass** die Membran (7) wasserdampfdurchlässig ausgeführt ist.

9. Sonde (1) zur Messung des Sauerstoffgehaltes in biologischem Material
- mit mindestens einer optischen Faser (2), welche proximal an eine Lichtquelle einerseits und an einen Lichtsensor andererseits optisch ankoppelbar ist,
- mit einem sauerstoffsensitiven Farbstoff (5),
**dadurch gekennzeichnet, dass** ein distaler Faserabschnitt (6) einschließlich der distalen Endfläche (4) umschlossen ist von einer sauerstoffdurchlässigen, flüssigkeitsundurchlässigen Membran (7), die im umschlossenen Bereich einen die distale Endfläche (4) umgebenden Gasraum (8) vorgibt, wobei der Farbstoff (5)
- als Beschichtung auf der den Gasraum (8) begrenzenden Membran (7) vorgesehen ist oder in mindestens einen Abschnitt einer Wand der Membran (7) eingearbeitet ist.

10. Katheter (14)
- mit einer Sonde (1) nach einem der Ansprüche 1 bis 9,
- mit einem Temperatursensor (30) zur Messung der Temperatur des den Katheter umgebenden biologischen Materials,
- sowie vorzugsweise mit einem Drucksensor (28) zur Messung des Drucks im den Katheter (14) umgebenden biologischen Materials.

11. Katheter nach Anspruch 10, **dadurch gekennzeichnet, dass** der Temperatursensor (30) zumindest abschnittsweise im Gasraum (8) angeordnet ist.

12. Katheter nach Anspruch 10 oder 11 unter Rückbeziehung auf Anspruch 4, **dadurch gekennzeichnet, dass** eine Katheterspitze (19; 32) die distale Abdichtung des Membranschlauches (11) der Membran (7) darstellt.

## Claims

1. Probe (1) for measuring the oxygen content in biological material
- with at least one optical fibre (2) which can be proximally optically coupled to a light source via one end, and to a light sensor via the other,
- with an oxygen-sensitive dye (5) which is arranged at a distal end face (4) of the fibre (2) and is optically coupled thereto,
**characterised in that** a distal fibre portion (6), including the distal end face (4) together with the dye (5), is enclosed by an oxygen-permeable, liquid-impermeable membrane (7) which, in the enclosed region, defines a gas compartment (8) enclosing the distal end face (4) with the dye (5), the dye (5)
- being provided as a coating on the distal end face (4).

2. Probe according to claim 1, **characterised in that** the thickness of the membrane (7) is uniform where it defines the gas compartment (8).

3. Probe according to claim 1 or 2, **characterised in that** the gas compartment (8) is, at least in portions, in the form of a cylinder, the longitudinal axis (9) of which is parallel to or coincides with the fibre axis (10) in the distal fibre portion (6).

4. Probe according to any one of claims 1 to 3, **characterised in that** the membrane (7) comprises a membrane tube (11), the ends (12, 13) of which are sealed against penetration of liquid for defining the gas compartment (8).

5. Probe according to any one of claims 1 to 4, **characterised in that** the membrane (7) is formed from one of the materials: silicone rubber, PE, PTFE or FEP.

6. Probe according to any one of claims 1 to 5, **characterised in that** the membrane (7) is sufficiently flexible to be deformable under the influence of a gas pressure in the gas compartment (8).

7. Probe according to any one of claims 1 to 6, **characterised in that** the gas compartment (8) is filled with air before insertion of the probe (1).

8. Probe according to any one claims 1 to 7, **characterised in that** the membrane (7) is configured to be water vapour-permeable.

9. Probe (1) for measuring the oxygen content in biological material
- with at least one optical fibre (2) which can be proximally optically coupled to a light source via one end, and to a light sensor via the other,
- with an oxygen-sensitive dye (5),
**characterised in that** a distal fibre portion (6), including the distal end face (4), is enclosed by an oxygen-permeable, liquid-impermeable membrane (7) which, in the enclosed region, defines a gas compartment (8) enclosing the distal end face (4) with the dye (5), the dye (5)
- being provided as a coating on the membrane (7) delimiting the gas compartment (8) or being incorporated into it least a portion of a wall of the membrane (7).

10. Catheter (14)
- with a probe (1) according to any one of claims 1 to 9,
- with a temperature sensor (30) for measuring the temperature of the biological material surrounding the catheter,
- and preferably comprising a pressure sensor (28) for measuring the pressure in the biological material surrounding the catheter (14).

11. Catheter according to claim 10, **characterised in that** the temperature sensor (30) is arranged, at least in part, in the gas compartment (8).

12. Catheter according to claim 10 or 11 with reference to claim 4, **characterised in that** a catheter tip (19; 32) represents the distal sealing of the membrane tube (11) of the membrane (7).

## Revendications

1. Sonde (1), destinée à mesurer la teneur en oxygène dans un matériau biologique,
- comportant au moins une fibre (2) optique, qui peut être couplée par voie optique en proximal, d'une part, à une source lumineuse et, d'autre part, à un capteur de lumière,
- comportant un colorant (5) sensible à l'oxygène, qui est agencé au niveau d'une surface d'extrémité distale (4) de la fibre (2) et qui est couplé optiquement à celle-ci,
**caractérisée en ce qu'**un tronçon de fibre (6) distal, y compris la surface d'extrémité distale (4) avec le colorant (5), est entouré par une membrane (7) perméable à l'oxygène, imperméable aux liquides, qui, dans la zone entourée, définit une chambre de gaz (8) entourant la surface d'extrémité distale (4) avec le colorant (5),
- ledit colorant (5) étant prévu sous forme de revêtement sur la surface d'extrémité distale (4).

2. Sonde selon la revendication 1, **caractérisée en ce que** l'épaisseur de la membrane (7) est uniforme là où elle définit la chambre de gaz (8).

3. Sonde selon la revendication 1 ou 2, **caractérisée en ce que** la chambre de gaz (8) a au moins par zones la forme d'un cylindre, dont l'axe longitudinal (9) est parallèle à l'axe (10) de la fibre dans le tronçon de fibre (6) distal ou coïncide avec ledit axe.

4. Sonde selon l'une quelconque des revendications 1 à 3, **caractérisée en ce que** la membrane (7) comporte un tuyau flexible (11) formant membrane dont les extrémités (12, 13) sont rendues étanches à toute pénétration de liquide en vue de définir la chambre de gaz (8).

5. Sonde selon l'une quelconque des revendications 1 à 4, **caractérisée en ce que** la membrane (7) est formée par l'un des matériaux : caoutchouc siliconé, PE, PTFE ou FEP.

6. Sonde selon l'une quelconque des revendications 1 à 5, **caractérisée en ce que** la membrane (7) est flexible de telle sorte qu'elle est déformable sous l'effet d'une pression du gaz, présente dans la chambre de gaz (8).

7. Sonde selon l'une quelconque des revendications 1 à 6, **caractérisée en ce que** la chambre de gaz (8) est remplie d'air avant l'utilisation de la sonde (1).

8. Sonde selon l'une quelconque des revendications 1 à 7, **caractérisée en ce que** la membrane (7) est réalisée perméable à la vapeur d'eau.

9. Sonde (1), destinée à mesurer la teneur en oxygène dans un matériau biologique,
- comportant au moins une fibre (2) optique, qui peut être couplée par voie optique en proximal, d'une part, à une source lumineuse et, d'autre part, à un capteur de lumière,
- comportant un colorant (5) sensible à l'oxygène,
**caractérisée en ce qu'**un tronçon de fibre (6) distal, y compris la surface d'extrémité distale (4), est entouré par une membrane (7) perméable à l'oxygène, imperméable aux liquides, qui, dans la zone entourée, définit une chambre de gaz (8) entourant la surface d'extrémité distale (4),
- ledit colorant (5) étant prévu sous forme de revêtement sur la membrane (7) délimitant la chambre de gaz (8) ou étant inséré dans au moins une partie d'une paroi de la membrane (7).

10. Cathéter (14)
- avec une sonde (1) selon l'une quelconque des revendications 1 à 9,
- avec un capteur de température (30) destiné à mesurer la température du matériau biologique entourant le cathéter,
- ainsi que, de préférence, avec un capteur de pression (28) destiné à mesurer la pression dans le matériau biologique entourant le cathéter (14).

11. Cathéter selon la revendication 10, **caractérisé en ce que** le capteur de température (30) est agencé au moins par zones dans la chambre de gaz (8).

12. Cathéter selon la revendication 10 ou 11 avec renvoi à la revendication 4, **caractérisé en ce qu'**une pointe de cathéter (19 ; 32) constitue l'étanchéité distale du tuyau flexible (11) de la membrane (7).
